(19) Europäisches Patentamt
European Patent Office
Office européen des brevets

(11) Veröffentlichungsnummer: **0 156 278 B1**

(12) **EUROPÄISCHE PATENTSCHRIFT**

(45) Veröffentlichungstag der Patentschrift: **02.03.88**

(21) Anmeldenummer: **85103100.5**

(22) Anmeldetag: **18.03.85**

(51) Int. Cl.⁴: **C 07 C 49/813**, C 07 C 49/84, C 07 C 45/28, C 07 C 45/36, C 07 C 97/10, C 07 D319/20, C 07 D317/46

# (54) Verfahren zur Herstellung von mit Fluor enthaltenden Gruppen substituierten, symmetrischen Benzophenonen, deren Verwendung und neue, mit Fluor enthaltenden Gruppen substituierte, symmetrische Benzophenone.

(30) Priorität: **28.03.84 DE 3411326**

(43) Veröffentlichungstag der Anmeldung: **02.10.85 Patentblatt 85/40**

(45) Bekanntmachung des Hinweises auf die Patenterteilung: **02.03.88 Patentblatt 88/09**

(84) Benannte Vertragsstaaten: **CH DE FR GB IT LI**

(56) Entgegenhaltungen:
**EP-A- 0 004 710**
**GB-A- 446 450**
**US-A- 3 732 307**
**US-A- 4 120 687**
**CHEMICAL ABSTRACTS, Band 80, 1974, Seite 85, Nr. 134887e, Columbus, Ohio, US; M.I. DRONKINA et al.: "Triphenylmethane dyes containing bis(trifluoromethyl)amino groups" & ZH. ORG. KHIM. 1973, 9(10), 2167-72**
**CHEMISTRY LETTERS, Nr. 6, Juni 1984, Seiten 881-884, The Chemical Society of Japan; K. TAKUMA et al.: "An effect of trifluoromethyl group on photosensitizing behavior of benzophenone derivatives"**
**Die Akte enthält technische Angaben, die nach dem Eingang der Anmeldung eingereicht wurden und die nicht in dieser Patentschrift enthalten sind.**

(73) Patentinhaber: **BAYER AG**
**Konzernverwaltung RP Patentabteilung**
**D-5090 Leverkusen 1 Bayerwerk (DE)**

(72) Erfinder: **Marhold, Albrecht, Dr.**
**Carl-Duisberg-Strasse 329**
**D-5090 Leverkusen 1 (DE)**
Erfinder: **Klauke, Erich, Dr.**
**Eichendorffweg 8**
**D-5068 Odenthal (DE)**
Erfinder: **Kysela, Ernst, Dr.**
**Virchowstrasse 14**
**D-5060 Bergisch-Gladbach 2 (DE)**

**EP 0 156 278 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von mit Fluor enthaltenden Gruppen substituierten, symmetrischen Benzophenonen durch Umsetzung von mit Fluor enthaltenden Gruppen substituierten aromatischen Verbindungen mit Formaldehyd und/oder einem Formaldehydderivat unter Zusatz von Fluorwasserstoff, Fluorsulfonsäure und/oder Schwefelsäure zu mit Fluor enthaltenden Gruppen substituierten, symmetrischen Diphenylmethanen und deren Oxidation, sowie die Verwendung solcher Benzophenone zur Herstellung von Polyetherketonen und neue, mit Fluor enthaltenden Gruppen substituierte, symmetrische Benzophenone.

Es ist beschrieben, daß die Umsetzung von aromatischen Verbindungen, z. B. Benzotrifluorid, mit Paraformaldehyd und Chlorsulfonsäure zu den entsprechenden chlormethylierten Aromaten, z. B. zu 3-Trifluormethylbenzylchlorid, führt (siehe US-PS 3 465 051). Eine Kondensation von zwei Molekülen der aromatischen Ausgangsverbindung zu den entsprechenden Diphenylmethanen ist dabei nicht beobachtet worden.

Die Herstellung von einigen mit Fluor enthaltenden Gruppen substituierten Diphenylmethanen aus aromatischen, mit Fluor enthaltenden Gruppen substituierten Verbindungen und mit Fluor enthaltenden Gruppen substituierten Alkylierungsmitteln in Gegenwart von Fluorwasserstoff, z. B. die Herstellung von Bis-(3-Trifluormethyl-phenyl)-methan aus Trifluormethyl-benzyl-methylether und Benzotrifluorid, ist in der DE-OS 31 51 365 beschrieben. Dieses Verfahren hat den Nachteil, daß auch für die Herstellung von symmetrischen, mit Fluor enthaltenden Gruppen substituierten Diphenylmethanen zwei unterschiedlich substituierte aromatische Ausgangsverbindungen eingesetzt werden müssen.

Aus der Gruppe der mit Fluor enthaltenden Gruppen substituierten, symmetrischen Benzophenone sind bisher nur zwei Verbindungen bekannt, nämlich das 3,3'-Bistrifluormethyl-benzophenon, das aus m-Trifluormethyl-phenyl-magnesiumbromid durch Umsetzung mit m-Trifluormethyl-benzonitril erhalten wurde (siehe Bull. Soc. Chim. France 1962, 587-93). Auch hier sind für die Herstellung des symmetrischen 3,3'-Bistrifluormethyl-benzophenons zwei unterschiedlich substituierte aromatische Ausgangsverbindungen einzusetzen. Außerdem ist die hier durchzuführende Grignard-Reaktion für eine technische Herstellung nicht geeignet. Bezüglich der Verwendbarkeit des 3,3'-Bistrifluormethyl-benzophenons ist aus der gleichen Literaturstelle bekannt, daß man daraus 3,3'-Bistrifluormethyl-benzhydrol, 3-Trifluormethyl-benzhydrol und 3,3',3'',3'''-Tetrakis-trifluormethyl-benzopinacol herstellen kann.

Außerdem ist aus US-A-3 732 307, Beispiel 7 die Verbindung 4,4'-Bistrifluormethyl-benzophenon bekannt.

Weiterhin ist aus GB-A-446 450 ein Verfahren zur Herstellung von Diarylmethanen bekannt, bei dem Benzol, oder, im Gegensatz zur vorliegenden Erfindung, mit elektronenliefernden Substituenten substituierte Benzole mit Formaldehyd kondensiert werden. Bei dem Verfahren dieser GB-A-446 450 ist außerdem die Gegenwart von Alkohol ein wesentliches Merkmal.

Schließlich ist aus EP-A-4 710 4,4'-Difluorbenzophenon bekannt, bei dem es sich ebenfalls um eine Verbindung mit deaktivierenden Substituenten handelt. Die Herstellung erfolgt ausgehend von 4,4'-Diaminodiphenylmethan.

Es wurde nun ein Verfahren zur Herstellung von mit Fluor enthaltenden Gruppen substituierten, symmetrischen Benzophenonen der Formel

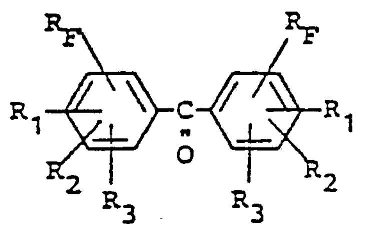

(I)

in der

$R_F$ für $CF_3$, $CF_2CF_3$, $CF_2CCl_3$, $OCF_3$, $OCF_2Cl$, $OCF_2CF_2H$, $OCF_2CHFCl$, $OCF_2CCl_3$, $SCF_3$, $SCF_2Cl$, $SCF_2CF_3$, $SCF_2CF_2H$, $SCF_2CHFCl$ oder $N(CF_3)_2$ und $R_1$ für Wasserstoff stehen oder

$R_F$ und $R_1$ gemeinsam für $—OCF_2O—$, $—OCF_2CF_2O—$, $—OCF_2CHFO—$ oder $—OCF_2CFClO—$ stehen und

$R_2$ für Wasserstoff, Halogen, eine Alkyl-, Alkoxy-, Alkylthio- oder für eine durch Halogen substituierte Alkoxy- oder Alkylthiogruppe steht und

$R_3$ für Wasserstoff, Halogen oder eine Alkylgruppe steht,

gefunden, das dadurch gekennzeichnet ist, daß man mit Fluor enthaltenden Gruppen substituierte aromatische Verbindungen der Formel

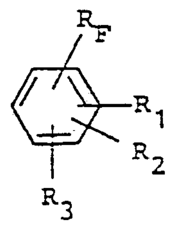

(II)

in der $R_F$, $R_1$, $R_2$ und $R_3$ die oben angegebene Bedeutung haben, mit Formaldehyd und/oder einem Formaldehydderivat unter Zusatz von Fluorwasserstoff, Fluorsulfonsäure und/oder Schwefelsäure zu mit Fluor enthaltenden Gruppen substituierten, symmetrischen Diphenylmethanen der Formel

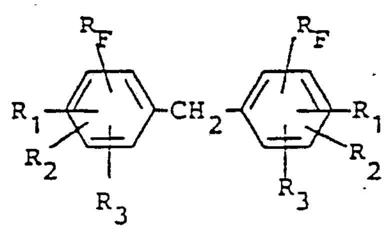

(III)

in der $R_F$, $R_1$, $R_2$ und $R_3$ die oben angegebene Bedeutung haben, umsetzt und diese Diphenylmethane oxidiert.

Sofern in den Formeln (I), (II) und (III) $R_2$ und/oder $R_3$ für Halogen oder für eine durch Halogen substituierte Alkoxy- oder Alkylthiogruppe steht, steht Halogen vorzugsweise für Fluor und/oder Chlor. Sofern in den Formeln (I), (II) und (III) $R_2$ und/oder $R_3$ für eine Alkyl-, Alkoxy-, Alkylthio- oder eine durch Halogen substituierte Alkoxy- oder Alkylthiogruppe steht, enthalten diese Gruppen vorzugsweise 1 bis 6, besonders bevorzugt 1 bis 4 C-Atome.

Die in das erfindungsgemäße Verfahren einzusetzenden aromatischen Verbindungen der Formel (II) sind beispielsweise gemäß der EP-OS 0 008 453, gemäß der EP-OS 0 011 179 oder gemäß J. Org. Chem. 44, 2907 (1979) oder auf dazu analoge Weise erhältlich.

Vorzugsweise werden in das erfindungsgemäße Verfahren Verbindungen der Formel (II) eingesetzt, bei denen

$R_F$ für $CF_3$, $OCF_3$, $OCF_2Cl$ oder $SCF_3$ und $R_1$ für Wasserstoff stehen oder

$R_F$ und $R_1$ gemeinsam für $—OCF_2CFClO—$, $—OCF_2O—$ oder $—OCF_2CHFO—$ stehen,

$R_2$ für Wasserstoff, Fluor, Chlor oder $C_1$- bis $C_4$-Alkyl steht und

$R_3$ für Wasserstoff oder Chlor steht.

Besonders bevorzugt werden in das erfindungsgemäße Verfahren Benzotrifluorid, Chlorbenzotrifluoride, Fluorbenzotrifluoride, 2-Chlor-5-methylbenzotrifluorid, Trifluormethoxybenzol, Difluorchlormethoxybenzol, 2,2-Difluorbenzodioxol, 2-Chlor-2,3,3-trifluorbenzodioxen und 2,3,3-Trifluorbenzodioxen eingesetzt.

Formaldehyd kann beispielsweise gasförmig, in Form eines Oligomeren (z. B. als Trioxan) und/oder als Polymer (z. B. als Polyoxymethylen) eingesetzt werden. Als Formaldehydderivate kommen beispielsweise Halogenmethylarylether, Halogenmethylarylthioether, Chlormethylisocyanat, Urotropin (= Hexamethylentetramin), Methoxymethylisocyanat oder analoge Verbindungen in Frage. Formaldehyd einer bestimmten Form und Formaldehydderivate können jeweils für sich alleine oder in beliebigen Mischungen untereinander eingesetzt werden. Bevorzugt ist der Einsatz von Trioxan und Polyoxymethylen.

Formaldehyd und/oder Formaldehydderivate werden im allgemeinen mindestens in der stöchiometrisch erforderlichen Menge eingesetzt. Bevorzugt ist es, einen Überschuß an Formaldehyd und/oder Formaldehydderivaten einzusetzen, beispielsweise einen 0,3- bis 3-fachen molaren Überschuß.

Der Zusatz eines Kondensationsmittels bestehend aus Fluorwasserstoff, Fluorsulfonsäure und/oder Schwefelsäure kann in unterschiedlichen Mengen erfolgen. Beispielsweise sind Kondensationsmittelmengen von 30 bis 250 Gew.-%, bezogen auf eingesetzte Verbindung der Formel (II), geeignet. Vorzugsweise beträgt die Kondensationsmittelmenge von 50 bis 200 Gew.-%, bezogen auf eingesetzte Verbindung der Formel (II).

Von den Kondensationsmitteln sind Fluorwasserstoff und Fluorsulfonsäure bevorzugt.

Die Reihenfolge des Zusammenfügens von aromatischer Verbindung der Formel (II), Formaldehyd und/oder Formaldehydderivaten und Kondensationsmittel ist ohne besondere Bedeutung.

Die erfindungsgemäße Kondensationsreaktion kann im allgemeinen bei Normaldruck und bei Temperaturen von beispielsweise 0 bis 100 °C durchgeführt werden. Insbesondere, wenn als Kondensationsmittel Fluorwasserstoff eingesetzt wird und Temperaturen über 20 °C angewendet werden sollen, kann sie auch unter Druck durchgeführt werden.

Die erfindungsgemäße Kondensationsreaktion wird bevorzugt nur bis zu einem Umsatz von 50 bis 70 %, bezogen auf die eingesetzte Verbindung der Formel (II), durchgeführt.

Das Reaktionsgemisch aus der erfindungsgemäßen Kondensationsreaktion kann beispielsweise aufgearbeitet werden, indem man das Reaktionsgemisch auf Eiswasser austrägt, die sich bildende

organische Phase oder das sich bildenden organische Festprodukt abtrennt und diese fraktioniert destilliert, vorzugsweise im Vakuum oder umkristallisiert, z. B. aus Cyclohexan oder Methanol. Man kann nach dem Austragen des Reaktionsgemisches auf Eis oder Wasser auch die organischen Bestandteile mit einem Lösungsmittel aufnehmen, z. B. mit Dichlormethan, und aus dieser Lösung, gegebenenfalls nach Waschen und Trocknen der Lösung, das Produkt der Formel (III) gewinnen. Wenn Fluorwasserstoff als Kondensationsmittel eingesetzt wurde kann man auch so verfahren, daß man den Fluorwasserstoff bei Normaldruck und/oder vermindertem Druck durch Destillation entfernt und das verbleibende Gemisch fraktioniert destilliert, vorzugsweise im Vakuum. Die bei der Aufarbeitung gegebenenfalls abgetrennten unumgesetzten Anteile der aromatischen Verbindungen der Formel (II) können erneut in das erfindungsgemäße Verfahren eingesetzt werden.

Es ist nicht unbedingt erforderlich die Produkte der Formel (III) zu isolieren und/oder zu reinigen, jedoch werden in die folgende Oxidationsreaktion vorzugsweise möglichst reine Verbindungen der Formel (III) eingesetzt.

Die auf die vorbeschriebene Weise erhaltenen Diphenylmethanderivate der Formel (III) werden dann oxidiert. Diese Oxidation kann auf an sich bekannte Weise durchgeführt werden. Geeignete Oxidationsmittel zind z. B. Salpetersäure, Chromsäure, Chromate, Dichromate, Kaliumpermanganat, Mangandioxid, Sauerstoff, Luft, Selendioxid und Peroxide, wie Wasserstoffperoxid und Nickelperoxid. Bevorzugte Oxidationsmittel sind Salpetersäure, Sauerstoff und Chromverbindungen, in denen das Chrom in der Oxidationsstufe + 6 vorliegt. Die Oxidation wird vorzugsweise in einem sauren Medium und bei erhöhter Temperatur durchgeführt.

Die Reaktionsprodukte dieser Oxidation, d. h. die mit Fluor enthaltenden Gruppen substituierten, symmetrischen Benzophenone der Formel (I), können isoliert werden, indem man beispielsweise das Reaktionsgemisch auf Wasser oder Eiswasser austrägt, mit einem Extraktionsmittel, z. B. Dichlormethan extrahiert und aus dem Extrakt das Extraktionsmittel abzieht. Im Falle des Einsatzes von Salpetersäure als Oxidationsmittel kann man auch so verfahren, daß man das Reaktionsgemisch abkühlt und die dann vorliegenden festen Bestandteile durch Filtration oder Zentrifugieren abtrennt. Die so erhaltenen Benzophenone der Formel (I) können gegebenenfalls weiter gereinigt werden, beispielsweise durch Umkristallisation, z. B. aus Methanol.

Mit dem erfindungsgemäßen Verfahren werden mit Fluor enthaltenden Gruppen substituierte, symmetrische Benzophenone der Formel (I) auf einfache und auch zur Durchführung im technischen Maßstab geeignete Weise zugänglich gemacht. Im Hinblick auf den eingangs geschilderten Stand der Technik ist es ausgesprochen überraschend, daß mit der erfindungsgemäßen Kondensationsreaktion die mit Fluor enthaltenden Gruppen substituierten, symmetrischen Diphenylmethane der Formel (III) erhalten werden können. Außerdem war zu erwarten, daß das in der Kondensationsreaktion gebildete Wasser in der vorliegenden sauren Umgebung zur Abspaltung der Fluor enthaltenden Gruppen führt. Erst diese gute und einfache Zugänglichkeit dieser Diphenylmethane ermöglicht es, daraus die Benzophenone der Formel (I) herzustellen.

Die Benzophenone der Formel (I) können z. B. zur Herstellung neuartiger Polymere des Polyetherketon-Typs verwendet werden.

Die vorliegende Erfindung betrifft weiterhin neue, mit Fluor enthaltenden Gruppen substituierte, symmetrische Benzophenone der Formel

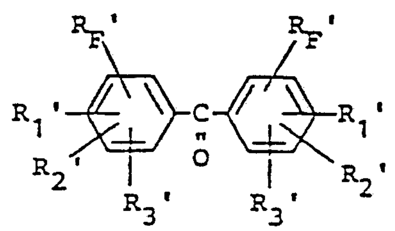

(IV)

in der

$R_F'$ für $CF_3$, $CF_2CF_3$, $CF_2CCl_3$, $OCF_3$, $OCF_2Cl$, $OCF_2CF_2H$, $OCF_2CHFCl$, $OCF_2CCl_3$, $SCF_3$, $SCF_2Cl$, $SCF_2CF_3$, $SCF_2CF_2H$ oder $SCF_2CHFCl$ und $R_1'$ für Wasserstoff stehen oder

$R_F'$ und $R_1'$ gemeinsam für $—OCF_2O—$, $—OCF_2CF_2O—$, $—OCF_2CHFO—$ oder $—OCF_2CFClO—$ stehen und

$R_2'$ für Wasserstoff, Halogen, eine Alkyl-, Alkoxy-, Alkylthio- oder für eine durch Halogen substituierte Alkoxy- oder Alkylthiogruppe steht und

$R_3'$ für Halogen oder eine Alkylgruppe steht.

Sofern in Formel (IV) $R_2'$ und/oder $R_3'$ für Halogen oder eine durch Halogen substituierte Alkoxy- oder Alkylthiogruppe steht, steht Halogen vorzugsweise für Fluor und/oder Chlor.

Sofern in Formel (IV) $R_2'$ und/oder $R_3'$ für eine Alkyl-, Alkoxy-, Alkylthio- oder eine durch Halogen substituierte Alkoxy- oder Alkylthiogruppe steht, enthalten diese Gruppen vorzugsweise 1 bis 6, besonders bevorzugt 1 bis 4 C-Atome.

Bevorzugte Verbindungen der Formel (IV) sind solche, bei denen

$R_F'$ für $CF_3$, $OCF_3$, $OCF_2Cl$ oder $SCF_3$ und $R_1'$ für Wasserstoff stehen oder
$R_F'$ und $R_1'$ gemeinsam für $—OCF_2O—$, $—OCF_2CF_2O—$, $—OCF_2CHFO—$ oder $—OCF_2CFClO—$ stehen und
$R_2'$ für Wasserstoff, Fluor, Chlor oder Methyl steht und
$R_3'$ für Fluor, Chlor oder Methyl steht.

Eine besonders bevorzugte Verbindung der Formel (IV) ist das 4,4'-Difluor-3,3'-bistrifluormethylbenzophenon.

Die vorliegende Erfindung betrifft weiterhin die Verwendung von mit Fluor enthaltenden Gruppen substituierten, symmetrischen Benzophenonen der Formel

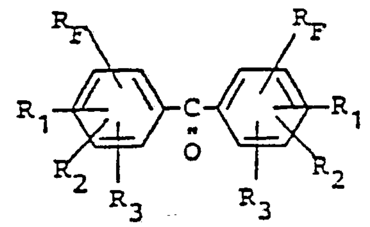

(I)

in der
$R_F$ für $CF_3$, $CF_2CF_3$, $CF_2CCl_3$, $OCF_3$, $OCF_2Cl$, $OCF_2CF_2H$, $OCF_2CHFCl$, $OCF_2CCl_3$, $SCF_3$, $SCF_2Cl$, $SCF_2CF_3$, $SCF_2CF_2H$, $SCF_2CHFCl$ oder $N(CF_3)_2$ und $R_1$ für Wasserstoff stehen oder
$R_F$ und $R_1$ gemeinsam für $—OCF_2O—$, $—OCF_2CF_2O—$, $—OCF_2CHFO—$ oder $—OCF_2CFClO—$ stehen und
$R_2$ für Wasserstoff, Halogen, eine Alkyl-, Alkoxy-, Alkylthio- oder für eine durch Halogen substituierte Alkoxy- oder Alkylthiogruppe steht und
$R_3$ für Wasserstoff, Halogen oder eine Alkylgruppe steht,
zur Herstellung von Polyetherketonen durch Umsetzung mit Diolen.

Bevorzugt für diese Verwendung sind Verbindungen der Formel (I), bei denen $R_F$ für $CF_3$, $R_1$ für Wasserstoff, $R_2$ für Fluor oder Chlor und $R_3$ für Wasserstoff stehen.

Als Diole kommen dabei beispielsweise Hydrochinon, 4,4'-Dihydroxydiphenylether, 4,4'-Dihydroxydiphenylsulfon und 4,4'-Dihydroxybenzophenon in Frage. Bevorzugt ist Hydrochinon.

Die Umsetzung von Verbindungen der Formel (I) mit Diolen erfolgt vorzugsweise unter Zusatz von Alkalien, z. B. Alkalihydroxiden oder Alkalihydriden, und in Gegenwart eines polaren Lösungsmittels, wie N-Methylpyrrolidon, Tetramethylensulfon oder Dimethylsulfoxid und bevorzugt unter Ausschluß von Feuchtigkeit oder Wasser.

Die so zugänglichen Polyetherketone können Molekulargewichte von über 20 000 aufweisen und zeichnen sich durch eine große Temperaturbeständigkeit aus.

Die folgenden Beispiele erläutern die vorliegende Erfindung, ohne sie in irgendeiner Weise zu beschränken.

## Beispiele

### Beispiel 1

In einem Reaktor aus Edelstahl wurden 200 ml Fluorwasserstoff bei 0 °C vorgelegt und nacheinander 146 g Benzotrifluorid und 30 g Trioxan zugegeben. Es wurde für 6 Stunden bei 20 °C gerührt. Danach wurde der Fluorwasserstoff abdestilliert, zuerst bei Normaldruck, dann im Vakuum. Anschließend wurde der verbleibende Rückstand einer fraktionierten Destillation unterworfen. Nachdem zunächst unumgesetztes Benzotrifluorid überging wurden 96 g 3,3'-Bistrifluormethyl-diphenylmethan mit einem Siedepunkt von 130 bis 140 °C bei 22 mbar und einem Schmelzpunkt von 39-40 °C erhalten.

### Beispiel 2

Es wurde gearbeitet wie in Beispiel 1, jedoch wurde anstelle von Trioxan 30 g polymerer Formaldehyd eingesetzt. Es wurden 66 g 3,3'-Bis-trifluormethyl-diphenylmethan erhalten.

### Beispiel 3

180 g 4-Chlorbenzotrifluorid und 39 g Paraformaldehyd wurden bei 20 °C vorgelegt und 1 Stunde gerührt. Dann wurden 100 g Fluorsulfonsäure im Verlaufe von 2 Stunden zugetropft. Nach 10-stündigem Rühren bei 20 °C wurde das Reaktionsgemisch auf Eis gegossen und die sich bildende organische Phase abgetrennt. Durch fraktionierte Destillation wurde zunächst unumgesetztes 4-Chlorbenzotrifluorid, anschließend 67 g 2,2'-Dichlor-5,5'-bistrifluormethyl-diphenylmethan mit einem Siedepunkt von 168 bis 173 °C bei 20 mbar und einem Schmelzpunkt von 32 bis 34 °C erhalten.

## Beispiel 4

Es wurde verfahren wie in Beispiel 3, jedoch wurden 60 g Paraformaldehyd eingesetzt und 105 g 2,2'-Dichlor-5,5'-bistrifluormethyl-diphenylmethan erhalten.

## Beispiel 5

In einen Reaktor aus Edelstahl wurden 90 g 2-Chlor-5-methyl-benzotrifluorid und 30 g Paraformaldehyd vorgelegt und bei 20 °C 100 ml Fluorsulfonsäure zugetropft. Nach 5-stündigem Rühren bei 20 °C wurde das Reaktionsgemisch auf Eis gegossen, die vorliegenden Festprodukte abgesaugt und aus Cyclohexan umkristallisiert. Es wurden 42 g 2,2'-Dichlor-3,3'-bistrifluormethyl-5,5'-dimethyl-diphenylmethan mit einem Schmelzpunkt von 118 bis 120 °C erhalten.

## Beispiel 6

180 g 2-Chlorbenzotrifluorid, 50 g Paraformaldehyd und 250 g Fluorsulfonsäure wurden analog der in Beispiel 5 beschriebenen Arbeitsweise umgesetzt. Durch Destillation wurden 145 g 4,4'-Dichlor-3,3'-bistrifluormethyldiphenylmethan, Siedepunkt 185 bis 190 °C/20 mbar, Schmelzpunkt 102 bis 104 °C aus Methanol, erhalten.

## Beispiel 7

In einer Rührapparatur aus Edelstahl wurden 900 g 2-Chlor-benzotrifluorid und 250 g Paraformaldehyd bei 10 °C vorgelegt. Dann wurden bei dieser Temperatur 700 g Fluorsulfonsäure zugetropft und für weitere 15 Stunden gerührt. Nach dem Austragen auf 2 000 g Eis wurde das organische Material in Dichlormethan aufgenommen, die organische Phase mit Wasser gewaschen, getrocknet und einer fraktionierten Destillation unterworfen. Neben Dichlormethan wurden zunächst 186 g unumgesetztes 2-Chlorbenzotrifluorid zurückgewonnen. Anschließend wurden 659 g 4,4'-Dichlor-3,3'-benzotrifluormethyl-diphenylmethan mit einem Siedepunkt von 180 bis 188 °C bei 16 mbar erhalten.

## Beispiel 8

In einem Edelstahlautoklaven wurden 84 g Urotropin, 300 ml Fluorwasserstoff und 97 g Trifluormethoxybenzol 5 Stunden lang auf 80 °C erhitzt. Nach dem Abdestillieren des Fluorwasserstoffs wurde der Rückstand auf Wasser gegeben, 30 Minuten lang gerührt, dann mit Dichlormethan extrahiert und anschließend die getrocknete organische Phase fraktioniert destilliert. Es wurden 11 g 4,4'-Bistrifluormethoxy-diphenylmethan mit einem Siedepunkt von 141 bis 143 °C bei 18 mbar erhalten.

## Beispiel 9

160 g 2-Methylbenzotrifluorid, 140 g Urotropin und 500 ml Fluorwasserstoff wurden entsprechend der in Beispiel 8 beschriebenen Arbeitsweise umgesetzt. Es wurden 45 g 4,4'-Dimethyl-3,3'-Bistrifluormethyl-phenylmethan, Schmelzpunkt 86-87 °C, Siedepunkt 135 bis 140 °C bei 0,15 mbar, erhalten.

## Beispiel 10

Es wurde verfahren wie in Beispiel 9, jedoch wurde anstelle von Urotropin 30 g Paraformaldehyd eingesetzt und bei 20 °C bearbeitet. Es wurden 133 g 4,4'-Dimethyl-3,3'-bistrifluormethyl-diphenylmethan mit einem Schmelzpunkt von 88 °C erhalten (Umkristallisiert aus Methanol).

## Beispiel 11

In einem Reaktionsgefäß aus Polyethylen wurden 360 g 4-Chlorbenzotrifluorid und 150 g Paraformaldehyd bei 20 °C vorgelegt und unter Rühren bei 20 °C 200 ml Fluorsulfonsäure zugetropft. Danach wurde 10 Stunden lang nachgerührt, anschließend 500 g Eis eingetragen und die organische Phase in Dichlormethan gelöst. Nach Abtrennen der organischen Phase wurde diese getrocknet und anschließend fraktioniert destilliert. Es wurden 108 g unumgesetztes 4-Chlorbenzotrifluorid erhalten, danach ein Zwischenlauf von 23 g, anschließend 200 g 2,2'-Dichlor-5,5'-bistrifluormethyl-diphenylmethan mit einem Siedepunkt von 130 bis 140 °C bei 0,45 mbar und ein Rückstand von 24 g, der zum größten Teil aus höher kondensierten Produkten bestand.

## Beispiel 12

Zu 2 000 ml 65 Gew.-%iger Salpetersäure wurden 390 g Bis-(4-Chlor-3-trifluormethyl-phenyl)-methan,

erhalten gemäß Beispiel 7, gegeben und 12 Stunden lang zum Sieden am Rückfluß erhitzt. Nach dem Abkühlen wurde das vorliegende Festprodukt abgesaugt (408 g) und aus Methanol umkristallisiert. Das erhaltene 4,4'-Dichlor-3,3'-bistrifluormethyl-benzophenon wies einen Schmelzpunkt von 98 bis 101 °C auf.

Beispiel 13

In eine Vorlage bestehend aus 60 g Natriumdichromat und 500 ml Essigsäure wurden 100 ml Schwefelsäure zugefügt. Dann wurden bei 45 °C 30 g Bis-(2-Chlor-5-trifluormethyl-phenyl)-methan, erhalten gemäß Beispiel 3, zugetropft. Es wurde 3 Stunden lang bei 45 bis 50 °C nachgerührt, dann abgekühlt und das Reaktionsgemisch in Wasser gegossen. Nach einer Extraktion mit Dichlormethan und Abziehen des Lösungsmittel wurden 22 g 2,2'-Dichlor-5,5'-bistrifluormethylbenzophenon mit einem Schmelzpunkt von 70 bis 72 °C erhalten.

Beispiel 14

Entsprechend der in Beispiel 12 beschriebenen Arbeitsweise wurden aus 60 g Bis-(4-Fluor-3-trifluormethyl-phenyl)-methan 54 g 4,4'-Difluor-3,3'-bistrifluormethyl-benzophenon mit einem Schmelzpunkt von 83 bis 84 °C erhalten.

Beispiel 15

Eine Mischung aus 50 g 2-Chlor-2,3,3-trifluorbenzodioxen, 20 g Trioxan und 100 ml Fluorwasserstoff wurde in einem Autoklaven für 5 Stunden auf 60 °C erhitzt. Anschließend wurde im Vakuum Fluorwasserstoff und unumgesetztes Ausgangsprodukt abgezogen und der verbleibende Rückstand destilliert. Es wurden 29 g Bis-(2-Chlor-2,3,3-trifluorbenzodioxenyl)-methan mit einem Siedepunkt von 150 bis 155 °C bei 0,1 mbar und einem Brechungsindex von $n_D^{20} = 1{,}509\,8$ erhalten.

Beispiel 16

In 300 ml trockenen N-Methyl-pyrrolidon wurden 12 g Hydrochinon mit 2,5 g Natriumhydrid umgesetzt und nach Ende der Salzbildung 36 g 4,4'-Difluor-3,3'-bistrifluormethyl-benzophenon zugesetzt. Dann wurde unter Feuchtigkeitausschluß 15 Stunden lang auf 150 °C erhitzt. Nach dem Abkühlen wurde das Lösungsmittel im Vakuum abdestilliert und der Rückstand mit Wasser gründlich gewaschen und anschließend getrocknet. Es wurde ein Polymer vom Polyetherketon-Typ mit Fluor enthaltenden Seitenketten erhalten, das einen Schmelzbereich von 250 bis 255 °C und ein Molekulargewicht von über 20 000 aufwies. Das Polymer zeichnete sich durch hohe Temperaturbeständigkeit aus.

**Patentansprüche**

1. Verfahren zur Herstellung von mit Fluor enthaltenden Gruppen subsituierten, symmetrischen Benzophenonen der Formel

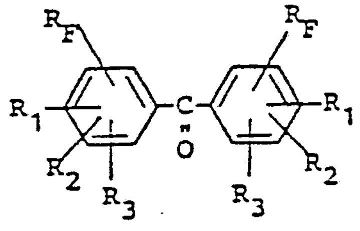

in der

$R_F$ für $CF_3$, $CF_2CF_3$, $CF_2CCl_3$, $OCF_3$, $OCF_2Cl$, $OCF_2CF_2H$, $OCF_2CHFCl$, $OCF_2CCl_3$, $SCF_3$, $SCF_2Cl$, $SCF_2CF_3$, $SCF_2CF_2H$, $SCF_2CHFCl$ oder $N(CF_3)_2$ und $R_1$ für Wasserstoff stehen oder

$R_F$ und $R_1$ gemeinsam für $-OCF_2O-$, $-OCF_2CF_2O-$, $-OCF_2CHFO-$ oder $-OCF_2CFClO-$ stehen und

$R_2$ für Wasserstoff, Halogen, eine Alkyl-, Alkoxy-, Alkylthio- oder für eine durch Halogen substituierte Alkoxy- oder Alkylthiogruppe steht und

$R_3$ für Wasserstoff, Halogen oder eine Alkylgruppe steht,

dadurch gekennzeichnet, daß man mit Fluor enthaltenden Gruppen substituierte aromatische Verbindungen der Formel

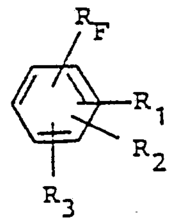

in der $R_F$, $R_1$, $R_2$ und $R_3$ die oben angegebene Bedeutung haben, mit Formaldehyd und/oder einem Formaldehydderivat unter Zusatz von Fluorwasserstoff, Fluorsulfonsäure und/oder Schwefelsäure zu mit Fluor enthaltenden Gruppen substituierten, symmetrischen Diphenylmethanen der Formel

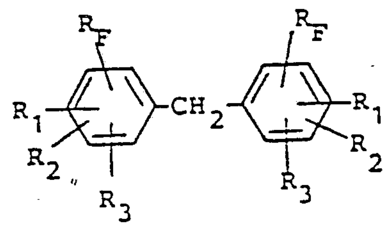

in der $R_F$, $R_1$, $R_2$ und $R_3$ die oben angegebene Bedeutung haben, umsetzt und diese Diphenylmethane oxidiert.

2. Verfahren nach Anspruch 1, dadurch gekennzeichnet, daß man mit Fluor enthaltenden Gruppen substituierte Verbindungen der angegebenen Formel einsetzt bei denen

$R_F$ für $CF_3$, $OCF_3$, $OCF_2Cl$ oder $SCF_3$ und $R_1$ für Wasserstoff stehen oder

$R_F$ und $R_1$ gemeinsam für $—OCF_2CFClO—$, $—OCF_2O—$ oder $—OCF_2CHFO—$ stehen,

$R_2$ für Wasserstoff, Fluor, Chlor oder $C_1$- bis $C_4$-Alkyl steht und

$R_3$ für Wasserstoff oder Chlor steht.

3. Verfahren nach Ansprüchen 1 und 2, dadurch gekennzeichnet, daß man Formaldehyd gasförmig, in Form eines Oligomeren und/oder in Form eines Polymeren und/oder als Formaldehydderivat Halogenmethylarylether, Halogenmethylarylthioether, Chlormethylisocyanat, Urotropin und/oder Methoxymethylisocyanat einsetzt.

4. Verfahren nach Ansprüchen 1 bis 3, dadurch gekennzeichnet, daß man Formaldehyd und/oder Formaldehydderivate in 0,3- bis 3-fachem molaren Überschuß einsetzt.

5. Verfahren nach Ansprüchen 1 bis 4, dadurch gekennzeichnet, daß man die Umsetzung zu Diphenylmethanen der angegebenen Formel nur bis zu einem Umsatz von 50 bis 70 %, bezogen auf die eingesetzte, mit Fluor enthaltenden Gruppen substituierte aromatische Verbindung, durchführt.

6. Verfahren nach Ansprüchen 1 bis 5, dadurch gekennzeichnet, daß man die Umsetzung zu Diphenylmethanen der angegebenen Formel bei Normaldruck und Temperaturen von 0 bis 100 °C durchführt.

7. Verfahren nach Ansprüchen 1 bis 6, dadurch gekennzeichnet, daß man mit Salpetersäure, Chromsäure, Chromaten, Dichromaten, Kaliumpermanganat, Mangandioxid, Sauerstoff, Luft, Selendioxid oder Peroxiden im sauren Medium und bei erhöhter Temperatur oxidiert.

8. Neue, mit Fluor enthaltenden Gruppen substituierte, symmetrische Benzophenone der Formel

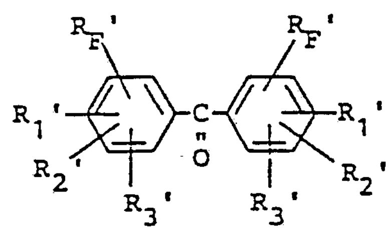

in der

$R_F'$ für $CF_3$, $CF_2CF_3$, $CF_2CCl_3$, $OCF_3$, $OCF_2Cl$, $OCF_2CF_2H$, $OCF_2CHFCl$, $OCF_2CCl_3$, $SCF_3$, $SCF_2Cl$, $SCF_2CF_3$, $SCF_2CF_2H$ oder $SCF_2CHFCl$ und $R_1'$ für Wasserstoff stehen oder

$R_F'$ und $R_1'$ gemeinsam für $—OCF_2O—$, $—OCF_2CF_2O—$, $—OCF_2CHFO—$ oder $—OCF_2CFClO—$ stehen und

$R_2'$ für Wasserstoff, Halogen, eine Alkyl-, Alkoxy-, Alkylthio- oder für eine durch Halogen substituierte Alkoxy- oder Alkylthiogruppe steht und

$R_3'$ für Halogen oder eine Alkylgruppe steht.

9. Neue Verbindungen gemäß Anspruch 8, dadurch gekennzeichnet, daß

$R_F'$ für $CF_3$, $OCF_3$, $OCF_2Cl$ oder $SCF_3$ und $R_1'$ für Wasserstoff stehen oder

$R_F'$ und $R_1'$ gemeinsam für $—OCF_2O—$, $—OCF_2CF_2O—$, $—OCF_2CHFO—$ oder $—OCF_2CFClO—$ stehen und

$R_2'$ für Wasserstoff, Fluor, Chlor oder Methyl steht und

$R_3'$ für Fluor, Chlor oder Methyl steht.

10. Verwendung von mit Fluor enthaltenden Gruppen substituierten, symmetrischen Benzophenonen der Formel

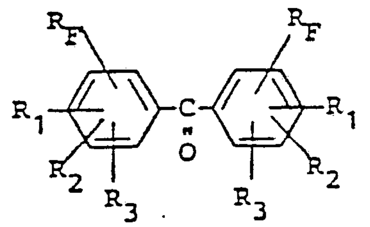

in der

$R_F$ für $CF_3$, $CF_2CF_3$, $CF_2CCl_3$, $OCF_3$, $OCF_2Cl$, $OCF_2CF_2H$, $OCF_2CHFCl$, $OCF_2CCl_3$, $SCF_3$, $SCF_2Cl$, $SCF_2CF_3$, $SCF_2CF_2H$, $SCF_2CHFCl$ oder $N(CF_3)_2$ und $R_1$ für Wasserstoff stehen oder

$R_F$ und $R_1$ gemeinsam für $—OCF_2O—$, $—OCF_2CF_2O—$, $—OCF_2CHFO—$ oder $—OCF_2CFClO—$ stehen und

$R_2$ für Wasserstoff, Halogen, eine Alkyl-, Alkoxy-, Alkylthio- oder für eine durch Halogen substituierte Alkoxy- oder Alkylthiogruppe steht und

$R_3$ für Wasserstoff, Halogen oder eine Alkylgruppe steht,

zur Herstellung von Polyetherketonen durch Umsetzung mit Diolen.

**Claims**

1. Process for the preparation of symmetric benzophenones substituted by groups containing fluorine, of the formula

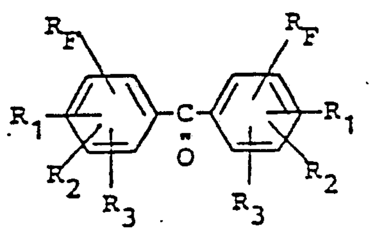

in which

$R_F$ represents $CF_3$, $CF_2CF_3$, $CF_2CCl_3$, $OCF_3$, $OCF_2Cl$, $OCF_2CF_2H$, $OCF_2CHFCl$, $OCF_2CCl_3$, $SCF_3$, $SCF_2Cl$, $SCF_2CF_3$, $SCF_2CF_2H$, $SCF_2CHFCl$ or $N(CF_3)_2$ and $R_1$ represents hydrogen, or

$R_F$ and $R_1$ together represent $—OCF_2O—$, $—OCF_2CF_2O—$, $—OCF_2CHFO—$ or $—OCF_2CFClO—$, and

$R_2$ represents hydrogen, halogen, an alkyl, alkoxy or alkylthio group or an alkoxy or alkylthio group which is substituted by halogen, and

$R_3$ represents hydrogen, halogen or an alkyl group,

characterised in that aromatic compounds substituted by groups containing fluorine, of the formula

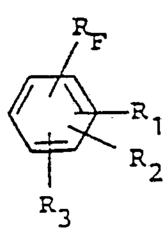

in which $R_F$, $R_1$, $R_2$ and $R_3$ have the abovementioned meaning, are reacted with formaldehyde and/or a formaldehyde derivative with the addition of hydrogen fluoride, fluorosulphonic acid and/or sulphuric acid, to give symmetric diphenylmethanes substituted by groups containing fluorine, of the formula

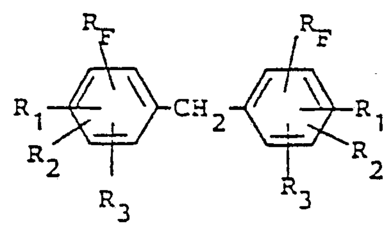

in which $R_F$, $R_1$, $R_2$ and $R_3$ have the abovementioned meaning, and these diphenylmethanes are oxidised.

2. Process according to Claim 1, characterised in that compounds substituted by groups containing fluorine, of the formula given, in which

$R_F$ represents $CF_3$, $OCF_3$, $OCF_2Cl$ or $SCF_3$ and $R_1$ represents hydrogen, or

$R_F$ and $R_1$ together represent $—OCF_2CFClO—$, $—OCF_2O—$ or $—OCF_2CHFO—$,

$R_2$ represents hydrogen, fluorine, chlorine or $C_1$- to $C_4$-alkyl and

$R_3$ represents hydrogen or chlorine,

are employed.

3. Process according to Claims 1 and 2, characterised in that formaldehyde is employed in gaseous form, in the form of an oligomer and/or in the form of a polymer, and/or halogenomethyl aryl ethers, halogenomethyl aryl thioethers, chloromethyl isocyanate, urotropine and/or methoxymethyl isocyanate are employed as the formaldehyde derivative.

4. Process according to Claims 1 to 3, characterised in that formaldehyde and/or formaldehyde derivatives are employed in a 0.3- to 3-fold molar excess.

5. Process according to Claims 1 to 4, characterised in that the reaction to give diphenylmethanes of the formula given is carried out only to a conversion of 50 to 70 %, based on the aromatic compound substituted by groups containing fluorine which is employed.

6. Process according to Claims 1 to 5, characterised in that the reaction to give diphenylmethanes of the formula given is carried out under normal pressure and at temperatures from 0 to 100 °C.

7. Process according to Claims 1 to 6, characterised in that the oxidation is carried out with nitric acid, chromic acid, chromates, dichromates, potassium permanganate, manganese dioxide, oxygen, air, selenium dioxide or peroxides in an acid medium and at an elevated temperature.

8. New symmetric benzophenones substituted by groups containing fluorine, of the formula

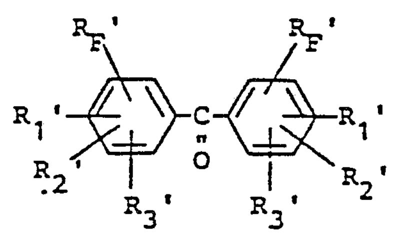

in which

$R_F'$ represents $CF_3$, $CF_2CF_3$, $CF_2CCl_3$, $OCF_3$, $OCF_2Cl$, $OCF_2CF_2H$, $OCF_2CHFCl$, $OCF_2CCl_3$, $SCF_3$, $SCF_2Cl$, $SCF_2CF_3$, $SCF_2CF_2H$ or $SCF_2CHFCl$ and $R_1'$ represents hydrogen, or

$R_F'$ and $R_1'$ together represent $—OCF_2O—$, $—OCF_2CF_2O—$, $—OCF_2CHFO—$ or $—OCF_2CFClO—$, and

$R_2'$ represents hydrogen, halogen, an alkyl, alkoxy or alkylthio group or an alkoxy or alkylthio group which is substituted by halogen, and

$R_3'$ represents halogen or an alkyl group.

9. New compounds according to Claim 8, characterised in that

$R_F'$ represents $CF_3$, $OCF_3$, $OCF_2Cl$ or $SCF_3$ and $R_1'$ represents hydrogen, or

$R_F'$ and $R_1'$ together represent $—OCF_2O—$, $—OCF_2CF_2O—$, $OCF_2CHFO—$ or $—OCF_2CFClO—$, and

$R_2'$ represents hydrogen, fluorine, chlorine or methyl and

$R_3'$ represents fluorine, chlorine or methyl.

10. Use of symmetric benzophenones substituted by groups containing fluorine, of the formula

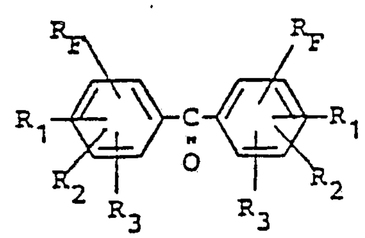

in which

$R_F$ represents $CF_3$, $CF_2CF_3$, $CF_2CCl_3$, $OCF_3$, $OCF_2Cl$, $OCF_2CF_2H$, $OCF_2CHFCl$, $OCF_2CCl_3$, $SCF_3$, $SCF_2Cl$, $SCF_2CF_3$, $SCF_2CF_2H$, $SCF_2CHFCl$ or $N(CF_3)_2$ and $R_1$ represents hydrogen, or

$R_F$ and $R_1$ together represent $-OCF_2O-$, $-OCF_2CF_2O-$, $-OCF_2CHFO-$ or $-OCF_2CFClO-$, and

$R_2$ represents hydrogen, halogen, and alkyl, alkoxy or alkylthio group or an alkoxy or alkylthio group which is substituted by halogen, and

$R_3$ represents hydrogen, halogen or an alkyl group,

for the preparation of polyether ketones by reaction with diols.

**Revendications**

1. Procédé de préparation de benzophénones symétriques substituées par des groupes contenant du fluor et répondant à la formule

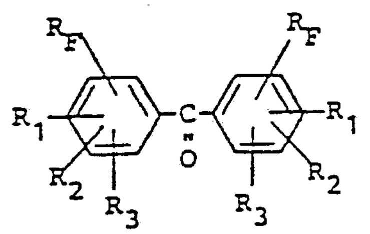

dans laquelle

$R_F$ représente $CF_3$, $CF_2CF_3$, $CF_2CCl_3$, $OCF_3$, $OCF_2Cl$, $OCF_2CF_2H$, $OCF_2CHFCl$, $OCF_2CCl_3$, $SCF_3$, $SCF_2Cl$, $SCF_2CF_3$, $SCF_2CF_2H$, $SCF_2CHFCl$ ou $N(CF_3)_2$ et $R_1$ représente l'hydrogène, ou bien

$R_F$ et $R_1$ forment ensemble un groupe $-OCF_2O-$, $-OCF_2CF_2O-$, $-OCF_2CHFO-$ ou $-OCF_2CFClO-$ et

$R_2$ représente l'hydrogène, un halogène, un groupe alkyle, alcoxy, alkylthio ou un groupe alcoxy ou alkylthio substitué par un halogène et

$R_3$ représente l'hydrogène, un halogène ou un groupe alkyle,

caractérisé en ce que l'on fait réagir des composés aromatiques substitués par des groupes contenant du fluor et répondant à la formule

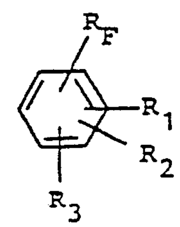

dans laquelle $R_F$, $R_1$, $R_2$ et $R_3$ ont les significations indiquées ci-dessus, avec le formaldéhyde et/ou un dérivé du formaldéhyde, avec adjonction de fluorure d'hydrogène, d'acide fluorosulfonique et/ou d'acide sulfurique, ce qui donne des diphénylméthanes symétriques substitués par des groupes contenant du fluor et répondant à la formule

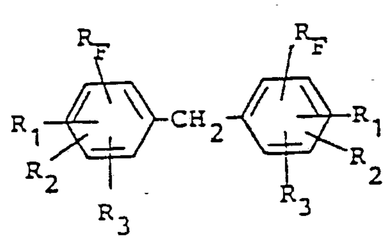

dans laquelle $R_F$, $R_1$, $R_2$ et $R_3$ ont les significations indiquées ci-dessus, qu'on soumet à oxydation.

2. Procédé selon la revendication 1, caractérisé en ce que l'on utilise des composés substitués par des groupes contenant du fluor et répondant à la formule indiquée, dans laquelle :

$R_F$ représente $CF_3$, $OCF_3$, $OCF_2Cl$ ou $SCF_3$ et $R_1$ représente l'hydrogène ou bien

$R_F$ et $R_1$ représentent ensemble un groupe $-OCF_2CFClO-$, $-OCF_2O-$ ou $-OCF_2CHFO-$,

$R_2$ représente l'hydrogène, le fluor, le chlore ou un groupe alkyle en $C_1$-$C_4$ et

$R_3$ représente l'hydrogène ou le chlore.

3. Procédé selon les revendications 1 et 2, caractérisé en ce que l'on met en œuvre le formaldéhyde à

l'état gazeux, à l'état d'oligomère et/ou à l'état de polymère et/ou à l'état de dérivé du formaldéhyde consistant en un éther halogénométhylarylique, un thioéther halogénométhylarylique, l'isocyanate de chlorométhyle, l'hexaméthylène-tétramine et/ou l'isocyanate de méthoxyméthyle.

4. Procédé selon les revendications 1 à 3, caractérisé en ce que l'on utilise le formaldéhyde et/ou les dérivés du formaldéhyde en excès de 0,3 à 3 moles par mole.

5. Procédé selon les revendications 1 à 4, caractérisé en ce que l'on ne poursuit la conversion en les diphénylméthanes répondant à la formule indiquée que jusqu'à un taux de conversion de 50 à 70 %, par rapport au composé aromatique substitué par des groupes contenant du fluor qui a été mis en œuvre.

6. Procédé selon les revendications 1 à 5, caractérisé en ce que la réaction conduisant aux diphénylméthanes répondant à la formule indiquée est effectuée à pression normale et à des températures de 0 à 100 °C.

7. Procédé selon les revendications 1 à 6, caractérisé en ce que l'on oxyde à l'aide de l'acide nitrique, de l'acide chromique, de chromates, de bichromates, du permanganate de potassium, du bioxyde de manganèse, de l'oxygène, de l'air, du dioxyde de sélénium ou de peroxydes en milieu acide et à chaud.

8. Nouvelles benzophénones symétriques substituées par des groupes contenant du fluor et répondant à la formule

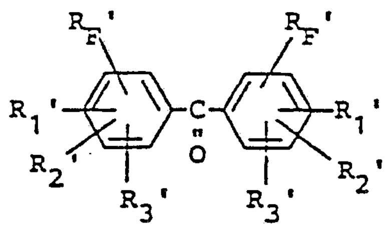

dans laquelle

$R_F'$ représente $CF_3$, $CF_2CF_3$, $CF_2CCl_3$, $OCF_3$, $OCF_2Cl$, $OCF_2CF_2H$, $OCF_2CHFCl$, $OCF_2CCl_3$, $SCF_3$, $SCF_2Cl$, $SCF_2CF_3$, $SCF_2CF_2H$ ou $SCF_2CHFCl$ et $R_1'$ représente l'hydrogène ou bien

$R_F'$ et $R_1'$ forment ensemble un groupe $—OCF_2O—$, $—OCF_2CF_2O—$, $—OCF_2CHFO—$ ou $—OCF_2CFClO—$ et

$R'_2$ représente l'hydrogène, un halogène, un groupe alkyle, alcoxy, alkylthio ou un groupe alcoxy ou alkylthio substitué par un halogène, et

$R_3'$ représente un halogène ou un groupe alkyle.

9. Nouveaux composés selon la revendication 8, caractérisés en ce que :

$R_F'$ représente $CF_3$, $OCF_3$, $OCF_2Cl$ ou $SCF_3$ et $R_1'$ représente l'hydrogène ou bien

$R_F'$ et $R_1'$ forment ensemble un groupe $—OCF_2O—$, $—OCF_2CF_2O—$, $—OCF_2CHFO—$ ou $—OCF_2CFClO—$ et

$R_2'$ représente l'hydrogène, le fluor, le chlore ou un groupe méthyle et

$R_3'$ représente le fluor, le chlore ou un groupe méthyle.

10. Utilisation des benzophénones symétriques substituées par des groupes contenant du fluor et répondant à la formule

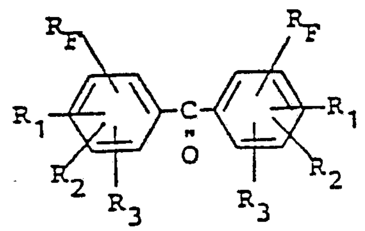

dans laquelle

$R_F$ représente $CF_3$, $CF_2CF_3$, $CF_2CCl_3$, $OCF_3$, $OCF_2Cl$, $OCF_2CF_2H$, $OCF_2CHFCl$, $OCF_2CCl_3$, $SCF_3$, $SCF_2Cl$, $SCF_2CF_3$, $SCF_2CF_2H$, $SCF_2CHFCl$ ou $N(CF_3)_2$ et $R_1$ représente l'hydrogène ou bien

$R_F$ et $R_1$ représentent ensemble un groupe $—OCF_2O—$, $—OCF_2CF_2O—$, $—OCF_2CHFO—$ ou $—OCF_2CFClO—$ et

$R_2$ représente l'hydrogène, un halogène, un groupe alkyle, alcoxy, alkylthio ou un groupe alcoxy ou alkylthio substitué par un halogène et

$R_3$ représente l'hydrogène, un halogène ou un groupe alkyle, pour la préparation de polyéthercétones par réaction avec des diols.